# EUROPÄISCHE PATENTSCHRIFT

(11) **EP 1 941 915 B1**
(45) Veröffentlichungstag und Bekanntmachung des Hinweises auf die Patenterteilung: **24.02.2010**
(21) Anmeldenummer: 07024340.7
(22) Anmeldetag: 14.12.2007
(51) Int. Cl.: A61L 9/12, B60H 3/00

(54) **Beduftungssystem, insbesondere für ein Kraftfahrzeug**
Fragrance system, in particular for a motor vehicle
Système de parfum, en particulier pour un véhicule automobile

(30) Priorität: 21.12.2006 DE 102006061825
(43) Veröffentlichungstag der Anmeldung: 09.07.2008
(73) Patentinhaber: Behr GmbH & Co. KG, 70469 Stuttgart (DE)
(72) Erfinder: Pitz, Eric, 70199 Stuttgart (DE); Lochmahr, Karl, 71665 Vaihingen (DE); Liermann, Andreas, 67117 Limburgerhof (DE); Boschert, Björn, 71254 Ditzingen (DE)

(56) Entgegenhaltungen:
- WO-A-03/028775
- GB-A- 2 401 047

## Beschreibung

Die Erfindung betrifft ein Beduftungssystem gemäß dem Oberbegriff des Anspruches 1.

Aus der DE 103 27 122 A1 ist eine Belüftungsvorrichtung, insbesondere zum Beduften eines Kraftfahrzeugs, bekannt, welche einen Duftstoff enthält, mit einer Dosiereinrichtung, wobei die Beduftungsvorrichtung zumindest einen Hohlkörper, der mit mindestens einer Öffnung versehen ist, und ein Element, insbesondere einen Hohlkörper, mit mindestens einer weiteren Öffnung umfasst. Dabei ist der Hohlkörper und/oder das Element mittels eines Motors derart antreibbar, dass eine Relativbewegung zwischen dem Hohlkörper und dem Element möglich ist, so dass die beiden Öffnungen in zumindest einer Stellung der Relativbewegung ein Austreten von Duftstoff von innen nach außen ermöglichen. Der Duftstoff selbst ist innerhalb des Hohlkörpers in einer Duftstoffpatrone oder -kartusche angeordnet, die bei Bedarf auswechselbar ist.

Eine andere Beduftungsvorrichtung ist aus der US 5,314,669 A bekannt. Bei dieser Beduftungsvorrichtung sind zwei in axialer Richtung zueinander angeordnete Hohlzylinder in einem im Wesentlichen als Hohlzylinder ausgebildeten Gehäuse drehbar aufgenommen. Der das Gehäuse bildende Hohlzylinder weist zwei rechteckförmige, sich in Längsrichtung erstreckenden Öffnungen in seiner Mantelfläche auf einander gegenüberliegenden Seiten auf, die sich jeweils zumindest über einen Teilbereich der inneren Hohlzylinder erstrecken. Die inneren Hohlzylinder weisen ebenfalls jeweils zwei einander gegenüberliegende Öffnungen in ihren Mantelflächen auf, die jedoch gestuft ausgebildet sind, d.h. die Form dreier jeweils um die Hälfte ihrer Länge versetzt zueinander angeordneter, langgestreckter und zusammen eine gemeinsame Öffnung bildender Rechtecke aufweisen. Die Öffnungen der inneren Hohlzylinder sind verdreht zueinander angeordnet, so dass verschiedene Beduftungsvariationen möglich sind.

Die US 5,178,327 A offenbart ein Beduftungssystem mit einem in mehrere Sektoren unterteilten, drehbar in einem Gehäuse angeordneten Hohlzylinder, wobei mindestens ein kuchenartiger Teil des Hohlzylinders in der Größe eines Segments fehlt, weshalb auf den Hohlzylinder im Folgenden als Teil-Hohlzylinder Bezug genommen wird. Die Segmente sind jeweils stirnseitig offen und in jedem der Segmente ist ein anderer Duftstoff eingelagert. Im Gehäuse ist auf einer oder beiden Seiten in axialer Richtung des Teil-Hohlzylinders eine in Ihrer Gestalt im Wesentlichen dem Querschnitt eines Segments entsprechende Öffnung ausgebildet. Um einen Raum zu beduften, wird das den gewünschten Duft enthaltende Segment vor die Öffnung gedreht. Wird keine Beduftung gewünscht, so wird das fehlende Segment vor die Öffnung gedreht. Um eine stärkere Beduftung eines Raumes zu ermöglichen, kann auch ein Lüfter vorgesehen sein, welches Luft durch die erste Öffnung in das Gehäuse und nach dem Durchströmen des Teil-Hohlzylinders durch die zweite, gegenüberliegende Öffnung dem zu beduftenden Raum zuführt.

Ferner ist aus der WO 03/028775 A1 eine Beduftungsvorrichtung mit einem Gebläse bekannt, welches Luft über einen oder zwei Duftstoffträger bläst. Um unterschiedliche Luftströmungsrichtungen zu realisieren, ist das Gebläse in unterschiedlichen Richtungen drehbar angeordnet.

Im Gegensatz dazu besteht bei der Beduftungsvorrichtung aus der GB 2 401 047 A die Möglichkeit durch Verstellung eines Verschlusselements unterschiedliche Strömungswege innerhalb der Beduftungsvorrichtung zu realisieren.

Derartige Beduftungssysteme lassen noch Wünsche offen.

Es ist Aufgabe der Erfindung, ein verbessertes Beduftungssystem zur Verfügung zu stellen.

Diese Aufgabe wird gelöst durch ein Beduftungssystem mit den Merkmalen des Anspruchs 1. Vorteilhafte Ausgestaltungen sind Gegenstand der Unteransprüche.

Hierbei ist ein Beduftungssystem, insbesondere für ein Kraftfahrzeug, vorgesehen, mit zumindest zwei Duftreservoirs, welche jeweils von einem Luftstrom von mit einem Duftstoff des entsprechenden Duftreservoirs zu versetzender Luft durchströmbar sind, mindestens einem Gebläse, welches den Luftstrom durch die Duftreservoirs bei Bedarf erzeugt, und einer Vorrichtung zur Einstellung des gewünschten Duftstoffs, mit dem die Luft zu versetzen ist, wobei die Vorrichtung zur Einstellung des gewünschten Duftstoffs durch das Gebläse in Verbindung mit einstellbaren Klappen, welche den Strömungsweg der Luft durch das Beduftungssystem bestimmen, gebildet ist. Die Öffnungen zu den einzelnen Duftreservoirs werden auf sehr einfache Weise durch eine (oder mehrere) Klappen freigegeben oder verschlossen, so dass eine bedarfsgerechte Beduftung möglich ist, ohne dass der nicht gewünschte Duftstoff in den Luftstrom gelangt.

Die Vorrichtung weist in Abhängigkeit von einer Betätigung des Gebläses einstellbare Klappen auf, wobei automatisch sich in Abhängigkeit der durch das Gebläse aufgebauten Luftströmung und der damit in Zusammenhang stehenden Druckverhältnisse einstellende Rückschlagklappen vorgesehen sind, so dass keine Aktuatoren für die Klappen erforderlich sind. Ein derartiges System hat einen sehr einfachen Aufbau und lässt sich sehr einfach über das Gebläse regeln, wobei neben den Beduftungszuständen bei entsprechender Ausgestaltung auch ein Zustand ohne Beduftung möglich ist, in welchem das Gebläse nicht betätigt wird, was insbesondere in Hinblick auf die allmähliche Gewöhnung an einen Duft bei konstanter Beduftung hilfreich ist.

Das Beduftungssystem weist vorzugsweise mindestens zwei Rückschlagklappen auf. Die Rückschlagklappen, insbesondere bevorzugt zumindest eine Mehrzahl der Rückschlagklappen des Beduftungssystems, werden besonders bevorzugt durch Gleichteile gebildet, so dass die Herstellungskosten gesenkt werden können.

Bevorzugt sind Rückschlagklappen vor den einzelnen Duftreservoirs angeordnet. Um den Bauraum zu optimieren, ragen im vollständig geöffneten Zustand der Rückschlagklappen die freien Enden derselben bis an das nachfolgend angeordnete Duftreservoir heran, ohne es jedoch zu berühren. Der vorhandene Bauraum bestimmt gegebenenfalls die Anzahl der nebeneinander angeordneten Rückschlagklappen.

Vorzugsweise sind mindestens zwei, insbesondere bevorzugt genau zwei, Rückschlagklappen im Bereich des Lufteintritts angeordnet und regeln zwei unterschiedliche Strömungswege der Luft durch das Beduftungssystem.

Mindestens eine Rückschlagklappe ist vorzugsweise im Bereich des Luftaustritts angeordnet und regelt zwei unterschiedliche Strömungswege der Luft durch das Beduftungssystem. Diese am Luftaustritt angeordnete Klappe verhindert ein Ausströmen des Duftstoffs aus dem nicht ausgewählten Duftreservoir und verhindert ein Einströmen des anderen, ausgewählten Duftstoffs in dieses Duftreservoir.

Diese Rückschlagklappe ist vorzugsweise in einer Mittelebene des Beduftungssystems angeordnet, wobei die Schwenkachse der Klappe gebläseseitig und das freie Ende der Klappe austrittsseitig angeordnet ist.

Das Gebläse kann vorzugsweise Luft in beide Richtungen fördern Hierfür weist es bevorzugt ein entsprechend ausgebildetes Laufrad auf, welches in beiden Drehrichtungen betrieben werden kann. Insbesondere bevorzugt ist die Drehrichtung des Laufrads rein elektronisch oder die elektrische Kontaktierung steuerbar, so dass das Gebläse einfach und ohne Mechanik regelbar ist.

Das Gebläse ist vorzugsweise in der Mittelebene des Beduftungssystems zwischen den Duftreservoirs angeordnet. Dies ermöglicht einen im Wesentlichen spiegelbildlichen Aufbau des gesamten Beduftungssystems bezüglich der Mittelebene.

Ein erfindungsgemäßes Beduftungssystem ist vorzugsweise Teil eines Kraftfahrzeug-Belüftungssystems und/oder einer Kraftfahrzeug-Klimaanlage. Hierbei ist besonders bevorzugt eine gemeinsame Ansteuerung mit dem Belüftungssystem und/oder der Klimaanlage für das Gebläse vorgesehen, so dass sich Anzahl der unabhängigen Steuerungen verringert. Besonders bevorzugt lassen sich auch andere Parameter, wie bspw. Fahrgeschwindigkeit, gewählte Temperatur, gewählte Gebläsestärke des Belüftungssystems oder Betriebsart des Belüftungssystems (wie Defrostbetrieb, Belüftung des Fußraums oder Belüftung über Mittel- und Seitendüsen), automatisch bei der Beduftung berücksichtigen, so dass sich automatisch eine optimierte Beduftung des Fahrzeuginnenraums ergibt.

Im Folgenden wird die Erfindung anhand von zwei Ausführungsbeispielen, teilweise unter Bezugnahme auf die Zeichnung, im Einzelnen erläutert. Es zeigen:
- Fig. 1: einen schematischen Schnitt quer durch ein Beduftungssystem gemäß dem ersten Ausführungsbeispiel mit zwei Duftreservoirs D1 und D2 in der Betriebsstellung "keine Beduftung",
- Fig. 2: eine Fig. 1 entsprechende Darstellung in der Stellung Beduftung mit dem im Duftreservoir D1 enthaltenen Duft 1, und
- Fig. 3: eine Fig. 1 entsprechende Darstellung in der Stellung Beduftung mit dem im Duftreservoir D2 enthaltenen Duft 2.

Ein vorliegend zentral in einer Instrumententafel eines Kraftfahrzeugs angeordnetes und in das Belüftungssystem des Kraftfahrzeugs integriertes Beduftungssystem 1 gemäß dem Ausführungsbeispiel weist ein Gehäuse 2 mit einem Gebläse 3, einer Mehrzahl von Rückschlagklappen 4 und zwei Duftreservoirs D1 und D2 auf, in denen Duftpatronen mit unterschiedlichen Duftstoffen angeordnet sind. Der Strömungsverlauf der das Beduftungssystem 1 durchströmenden Luft ist in der Zeichnung durch Pfeile angedeutet.

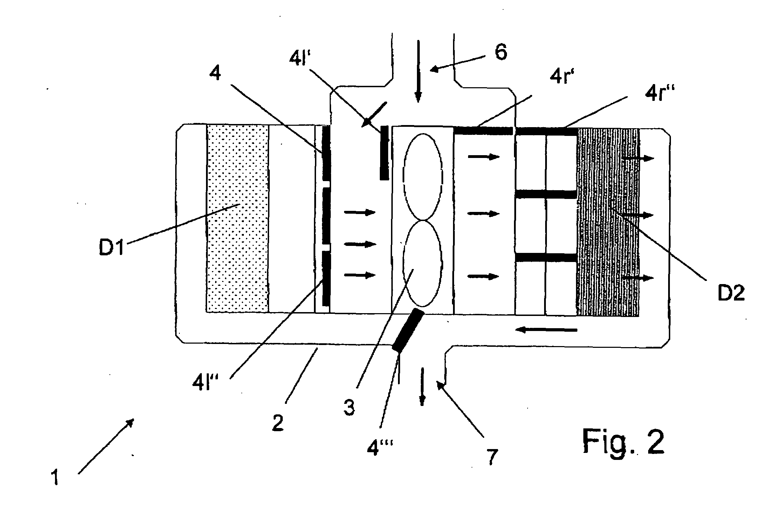

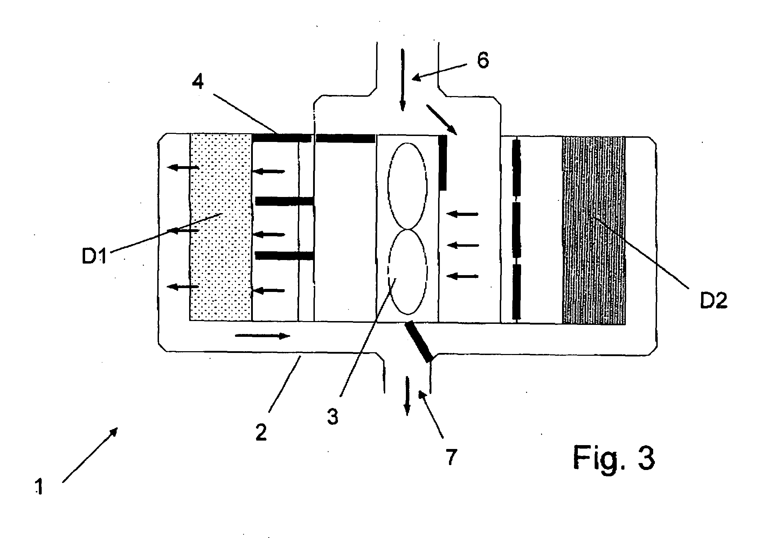

Das Laufrad des Gebläses 3 ist in einer Mittelebene des Beduftungssystems 1 angeordnet und weist eine Drehachse 5 auf, die senkrecht zur Ein- und Ausströmrichtung der Luft angeordnet ist und deren Verlängerung mittig durch die Duftreservoirs D1 und D2 verläuft. Das Laufrad ist derart ausgebildet, dass es in beiden Drehrichtungen betrieben werden kann, d.h. die von oben zugeführte (oder gegebenenfalls auch angesaugte) Luft kann sowohl gemäß der Darstellung von Fig. 2 von links nach rechts als auch gemäß der Darstellung von Fig. 3 von rechts nach links durch das Gebläse 3 strömen. Um ein Mitreißen von Luft aus dem jeweiligen Bereich des nicht benötigten Duftreservoirs zu verhindern, ist sind besagte Rückschlagklappen 4 vorgesehen. Sämtliche Rückschlagklappen 4 sind vorliegend um ihre Schwenkachsen um einen Winkel von ca. 90° verschwenkbar. Auf Grund der konstruktiven Ausgestaltung handelt es sich bei allen Klappen um Gleichteile.

Die Duftreservoirs D1 und D2 bestehen aus luftdurchlässigen, käfigartigen Patronen, vorliegend quaderförmig ausgebildet, in denen ein Duftstoff in lockerer Schüttung eingebracht ist.

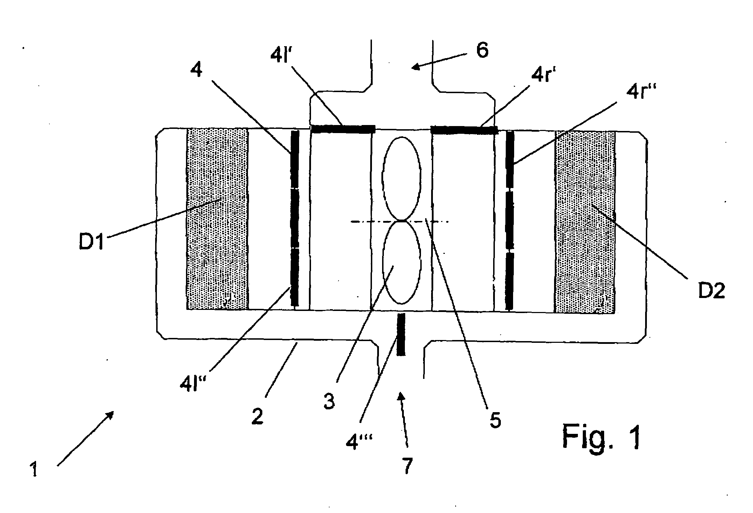

Die Funktion des Beduftungssystems 1 ist Folgende: Ausgehend vom Ruhezustand, der in Fig. 1 dargestellt ist, wird ein Luftstrom durch das Beduftungssystem 1 geleitet sowie das Gebläse 3 eingeschaltet, wobei das Gebläse 3 sich vorliegend derart dreht, dass Luft von links nach rechts gefördert wird (siehe Fig. 2). In Folge des Unterdrucks vor dem Gebläse 3 öffnet sich die linke obere Eintritts-Rückschlagklappe 41', während sich die rechte obere Eintritts-Rückschlagklappe 4r' in Folge des in Schließrichtung wirkenden Überdrucks automatisch schließt und damit ein Rückströmen der von dem Gebläse 3 geförderten Luft verhindert wird. Ferner wird von oben, durch die Eintrittsöffnung 6 Luft angesaugt. Die Duft-Rückschlagklappen 41", die zur Abgrenzung des Duftreservoirs D1 angeordnet sind, schließen sich in Folge des Unterdrucks, der vor denselben auf Grund der Saugkraft des Gebläses 3 herrscht, so dass das Duftreservoir D1 nicht oder nur durch vernachlässigbare Seitenströmungen von Luft durchströmt wird. Die Duft-Rückschlagklappen 4r" vor dem Duftreservoir D2 öffnen sich hingegen auf Grund des vom Gebläse 3 kommenden Luftstroms, so dass der Weg für die Luft durch das in seinen Seitenbereichen luftdurchlässige Duftreservoir D2 freigegeben ist. Nach dem Durchströmen des Duftreservoirs D2 gelangt die Luft in einen Luftkanal, welcher sie weiterleitet, vorliegend zuerst nach unten und anschließend wieder zurück zur Mittelebene des Beduftungssystems 1, in welcher die Auslassöffnung 7 fluchtend mit der Eintrittsöffnung 6 angeordnet ist. Die an der Auslassöffnung 7 mittig angeordnete Austritts-Rückschlagklappe 4''' wird nach links geschwenkt, so dass der Strömungsweg für die vom Duftreservoir D2 kommende Luft freigegeben ist, während der Strömungsweg von vom Duftreservoir D1 kommende Luft verschlossen ist.

Wird - in Folge einer Anforderung seitens eines Fahrzeuginsassen - nicht mehr der Duft des Duftreservoirs D2 sondern der Duft des Duftreservoirs D1 gewünscht, so wird die Drehrichtung des Gebläsemotors umgeschaltet, und das Gebläse 3 fördert Luft von recht nach links (siehe Fig. 3). In Folge der geänderten Strömungs- und Druckverhältnisse werden die linke obere Eintrits-Rückschlagkiappe 4l' und die rechten Duft-Rückschlagklappen 4r" geschlossen, während die rechte obere Eintritts-Rückschlagklappe 4r' und die linken Duft-Rückschlagklappen 4l" geöffnet werden. Die Austritts-Rückschlagklappe 4'" wird in ihre andere Endstellung bewegt.

Das Beduftungssystem 1 kann sowohl kontinuierlich als auch in einem Intervallbetrieb betrieben werden, bei dem einem ersten konstanten Zeitintervall mit Beduftung ein zweites konstantes Zeitintervall ohne Beduftung folgt, um einen Gewöhnungseffekt zu verhindern. Die Zeitspanne der Beduftung beträgt hierbei bevorzugt 5 bis 60 Sekunden, insbesondere bevorzugt ca. 10 Sekunden. Die Zeitspanne ohne Beduftung beträgt bevorzugt 30 Sekunden bis 2 Minuten, insbesondere bevorzugt ca. 50 bis 70 Sekunden und ganz besonders bevorzugt ca. 60 Sekunden. Die Regelung der Beduftung kann jedoch auch bedarfsorientiert erfolgen, wofür entsprechende Sensoren vorzusehen sind.

Die Intensität des gewünschten Dufts kann durch die Drehzahl des Gebläses und/oder das Verhältnis von "Laufzeit Gebläse" zu "Ruhezeit Gebläse" geregelt werden.

Gemäß einer Variante des ersten Ausführungsbeispiels entfallen die Eintritts-Rückschlagklappen. Der vom Gebläse bewirkte Luftstrom reißt hierbei einen Teil der direkt vom Lufteintritt kommenden Luft mit. Da diese nicht mit dem anderen, nicht ausgewählten Duftstoff in Berührung gekommen ist, ist dies unproblematisch. In Folge der zwei eingesparten Rückschlagklappen besteht ein Kostenvorteil, allerdings wird der obere Bereich der Duftreservoirs nicht in dem Maße durchströmt wird, wie der weiter unten, ausschließlich von vom Gebläse kommender Luft durchströmte Bereich.

Gemäß einem nicht in der Zeichnung dargestellten zweiten Ausführungsbeispiel sind zwei Gebläse mit vorgegebener Drehrichtung vorgesehen, die jeweils direkt vor den Duft-Rückschlagklappen angeordnet sind. Mittig ist ein zentraler Eintrittsluftkanal vorgesehen, da das in der Zeichnung dargestellte, zentrale Gebläse entfällt. Ferner entfallen die oberen Eintritts-Rückschlagklappen. Gegebenenfalls kann auch auf die Austritts-Rückschlagklappe verzichtet werden.

Das Vorsehen zweier getrennt ansteuerbarer Gebläse ermöglicht auch eine beliebige Mischung der beiden Düfte, sofern erwünscht. In diesem Fall ist jedoch eine Anordnung der Gebläse hinter den Duftreservoirs sinnvoll, so dass die anströmende Luft nicht so stark beeinträchtigt wird.

Das Beduftungssystem gemäß dem ersten und zweiten Ausführungsbeispiel muss nicht notwendigerweise in das Belüftungssystem des Kraftfahrzeugs integriert sein. Es sind auch "stand-alone" Lösungen möglich, wobei die Energie für das oder die Gebläse durch Batterien oder über einen Stecker von der Energieversorgung des Kraftfahrzeugs zur Verfügung gestellt werden kann. Die zu beduftende Luft kann direkt aus dem Fahrzeuginnenraum angesaugt werden.

## Patentansprüche

1. Beduftungssystem, insbesondere für ein Kraftfahrzeug, mit zumindest zwei Duftreservoirs (D1 und D2), welche jeweils von einem Luftstrom von mit einem Duftstoff des entsprechenden Duftreservoirs (D1 oder D2) zu versetzender Luft durchströmbar sind, mindestens einem Gebläse (3), welches den Luftstrom durch die Duftreservoirs (D1 und D2) bei Bedarf erzeugt, und einer Vorrichtung zur Einstellung des gewünschten Duftstoffs, mit dem die Luft zu versetzen ist, wobei die Vorrichtung zur Einstellung des gewünschten Duftstoffs durch das Gebläse (3) in Verbindung mit einstellbaren Klappen, welche den Strömungsweg der Luft durch das Beduftungssystem (1) bestimmen, gebildet ist, **dadurch gekennzeichnet, dass** die einstellbaren Klappen Rückschlagklappen (4) sind, die sich durch den sich bei Betätigung des Gebläses (3) einstellenden Strömungsverlauf der Luft und die Druckverhältnisse im Beduftungssystem (1) automatisch einstellen.

2. Beduftungssystem nach Anspruch 1, **dadurch gekennzeichnet, dass** das Beduftungssystem mindestens zwei Rückschlagklappen (4) aufweist.

3. Beduftungssystem nach Anspruch 1 oder 2, **dadurch gekennzeichnet, dass** die Rückschlagklappen (4) vor den Duftreservoirs (D1 und D2) angeordnet sind.

4. Beduftungssystem nach einem der Ansprüche 1 bis 3, **dadurch gekennzeichnet, dass** mindestens zwei Rückschlagklappen (4) im Bereich des Lufteintritts angeordnet sind und zwei unterschiedliche Strömungswege der Luft durch das Beduftungssystem (1) regeln.

5. Beduftungssystem nach einem der Ansprüche 1 bis 4, **dadurch gekennzeichnet, dass** mindestens eine Rückschlagklappe (4) im Bereich des Luftaustritts angeordnet ist und zwei unterschiedliche Strömungswege der Luft durch das Beduftungssystem (1) regelt.

6. Beduftungssystem nach einem der vorhergehenden Ansprüche, **dadurch gekennzeichnet, dass** das Gebläse (3) in beiden Drehrichtungen betätigbar ist.

7. Beduftungssystem nach einem der vorhergehenden Ansprüche, **dadurch gekennzeichnet, dass** das Gebläse (3) in der Mittelebene des Beduftungssystems (1) zwischen den Duftreservoirs (D1 und D2) angeordnet ist.

8. Kraftfahrzeug-Belüftungssystem und/oder -Klimaanlage, **gekennzeichnet durch** ein Beduftungssystem (1) nach einem der vorhergehenden Ansprüche.

9. Kraftfahrzeug-Belüftungssystem und/oder -Klimaanlage nach Anspruch 8, **dadurch gekennzeichnet, dass** das Gebläse (3) des Beduftungssystems (1) eine gemeinsame Ansteuerung mit dem Belüftungssystem und/oder der Klimaanlage aufweist.

## Claims

1. Fragrance system, especially for a motor vehicle, with at least two fragrance reservoirs (D1 and D2), which respectively can be flown through by an air flow to be infused with a fragrance of the corresponding fragrance reservoir (D 1 or D2), at least one fan (3), which generates the air flow through the fragrance reservoirs (D1 and D2) when necessary, and a device for adjusting the desired fragrance, with which the air is to be infused, wherein the device for adjusting the desired fragrance is formed by means of the fan (3) in connection with adjustable flaps which determine the flow path of the air through the fragrance system (1), **characterised in that** the adjustable flaps are check valves (4) which adjust automatically by means of the flow progression of the air which adjusts itself when actuating the fan (3) and the pressure ratios in the fragrance system (1).

2. Fragrance system according to claim 1, **characterised in that** the fragrance system comprises at least two check valves (4).

3. Fragrance system according to claim 1 or 2, **characterised in that** the check valves (4) are arranged in front of the fragrance reservoirs (D1 and D2)

4. Fragrance system according to one of claims 1 to 3, **characterised in that** at least two check valves (4) are arranged in the region of the air inlet and control two different flow paths of the air through the fragrance system (1).

5. Fragrance system according to one of claims 1 to 4, **characterised in that** at least one check valve (4) is arranged in the region of the air outlet and controls two different flow paths of the air through the fragrance system (1).

6. Fragrance system according to one of the preceding claims, **characterised in that** the fan (3) can be actuated in both directions of rotation.

7. Fragrance system according to one of the preceding claims, **characterised in that** the fan (3) is arranged in the centre plane of the fragrance system (1) between the fragrance reservoirs (D1 and D2).

8. Motor vehicle ventilation system and/or air conditioning unit, **characterised by** a fragrance system (1) according to one of the preceding claims.

9. Motor vehicle ventilation system and/or air conditioning unit according to claim 8, **characterised in that** the fan (3) of the fragrance system (1) comprises a common activation with the ventilation system and/or the air conditioning unit.

## Revendications

1. Système servant à parfumer, en particulier un véhicule à moteur, comprenant au moins deux réservoirs de parfum (D1 et D2) qui sont traversés respectivement par un flux d'air à remplacer par un parfum du réservoir de parfum correspondant (D1 ou D2), comprenant au moins une soufflante (3) qui, si nécessaire, produit le flux d'air à travers les réservoirs de parfum (D1 et D2), et comprenant un dispositif servant au réglage du parfum souhaité par lequel l'air est à remplacer, où le dispositif servant au réglage du parfum souhaité est formé par la soufflante (3), en association avec des clapets réglables qui déterminent la trajectoire d'écoulement de l'air à travers le système (1) servant à parfumer,
**caractérisé en ce que** les clapets réglables sont des clapets antiretour (4) qui se règlent automatiquement par le parcours d'écoulement de l'air se réglant lors de l'actionnement de la soufflante (3), et par les conditions de pression dans le système (1) servant à parfumer.

2. Système servant à parfumer selon la revendication 1, **caractérisé en ce que** le système servant à parfumer présente au moins deux clapets antiretour (4).

3. Système servant à parfumer selon la revendication 1 ou 2, **caractérisé en ce que** les clapets antiretour (4) sont disposés en amont des réservoirs de parfum (D1 et D2).

4. Système servant à parfumer selon l'une quelconque des revendications 1 à 3, **caractérisé en ce qu'**au moins deux clapets antiretour (4) sont disposés dans la zone de l'entrée d'air et règlent deux trajectoires différentes de l'écoulement de l'air à travers le système (1) servant à parfumer.

5. Système servant à parfumer selon l'une quelconque des revendications 1 à 4, **caractérisé en ce qu'**au moins un clapet antiretour (4) est disposé dans la zone de la sortie d'air et règle deux trajectoires différentes de l'écoulement de l'air à travers le système (1) servant à parfumer.

6. Système servant à parfumer selon l'une quelconque des revendications précédentes, **caractérisé en ce que** la soufflante (3) peut être actionnée dans les deux directions de rotation.

7. Système servant à parfumer selon l'une quelconque des revendications précédentes, **caractérisé en ce que** la soufflante (3) est disposée entre les réservoirs de parfum (D1 et D2), dans le plan médian du système (1) servant à parfumer.

8. Système de ventilation et / ou système de climatisation d'un véhicule à moteur, **caractérisé par** un système (1) servant à parfumer selon l'une quelconque des revendications précédentes.

9. Système de ventilation et / ou système de climatisation d'un véhicule à moteur selon la revendication 8, **caractérisé en ce que** la soufflante (3) du système (1) servant à parfumer présente une commande commune avec le système de ventilation et / ou avec le système de climatisation.
